# EUROPEAN PATENT APPLICATION

(11) **EP 4 696 265 A1**
(43) Date of publication of application: **18.02.2026**
(21) Application number: 24204582.1
(22) Date of filing: 04.10.2024
(51) Int. Cl.: A61B 18/18, A61B 18/00

(54) **LIGHT-BASED HAIR OR SKIN TREATMENT**

(30) Priority: 16.08.2024 WO PCT/CN2024/112840
(71) Applicant: Koninklijke Philips N.V., 5656 AG Eindhoven (NL)
(72) Inventor: TANG, Lijuan, 5656 AG Eindhoven (NL); LIN, Fu-Lung, 5656 AG Eindhoven (NL); GU, Jun, 5656 AG Eindhoven (NL)
(74) Representative: Philips Intellectual Property & Standards

(57) **Abstract**

A device is provided for hair removal or treatment of skin, comprising a light source for generating treatment light, an output window and a reflector for redirected output light from the light source to the output window. The reflector is segmented into at least two segments, wherein a gap is provided between the at least two segments. The gap is shielded from a direct light path from the light source by one of the segments. The gap promotes cooling of the light source.

## Description

### FIELD OF THE INVENTION

This invention relates to a device for light-based hair removal or treatment of skin.

### BACKGROUND OF THE INVENTION

For hair removal, light-based epilators are well-known. People use hair epilators or depilators to remove unwanted hair. Typical target areas for women are the face, armpit, arm, leg, bikini line, and body. Men use light-based epilators also on the chest and back.

Light based epilators typically use a flash lamp to generate the treatment light. The flash lamp operation generates heat, and this heat needs to be dissipated. However, the desire to direct all generated light to the treatment area generally results in a closed light reflecting chamber, and this makes it difficult to manage the heat generated by the flash lamp.

Excessive heat generation is both a safety concern and an efficiency issue, in that the light generation is more efficient at lower temperatures.

### SUMMARY OF THE INVENTION

The invention is defined by the claims.

According to examples in accordance with an aspect of the invention, there is provided a device for hair removal or treatment of skin, comprising:
a light source for generating treatment light;
an output window through which the treatment light is delivered to a user's skin, in use; and
a reflector for redirecting output light from the light source to the output window,
wherein the reflector is segmented into at least two segments, wherein a gap is provided between the at least two segments, wherein the gap is shielded from a direct light path from the light source by one of the segments.

In this design of hair removal device, the reflector used to direct light to the output window has a segmented design. There is a gap between at least two segments, and it functions to allow an airflow, and thereby promote cooling of the light source.

The gap is not in a direct path of light from the light source so that the reflection function is not disrupted. Instead, the gap is defined as an overlap region, wherein the inner segment acts as a light shield to the gap.

The reflector for example comprises at least three segments and at least two gaps between the segments. By having two gaps, a flow of air is enabled between the gaps and past the light source, such as a convection current.

The light source is for example a prism shape, for example cylindrical, with its length extending along a width direction of the output window. The segments each extend across the full width direction so that the gaps extend across the full length of the light source.

In one example, the reflector comprises an arcuate rear segment (around the back of the light source relative to the light output window), a top segment and a bottom segment. The gaps are thus at the top and bottom of the arcuate rear segment. The rear segment is the primary reflecting surface in that light is emitted from the light source in a rearward direction, and the rear segment reflects the light towards the output window. The rear segment is thus provided as a continuous reflecting surface, and the disruptions to the surface are provided at the top and bottom edges.

In a first design using these three segments, the arcuate rear segment extends partially around the light source, and the top and bottom segments outwardly overlap top and bottom edges of the arcuate rear segment. By "outwardly overlap" is meant that those segments are radially further away from the light source and there is an overlap rather than a gap between the segments in the circumferential direction. This design has a rear part nearer to the light source.

In a second design using these three segments, the arcuate rear segment extends partially around the light source, and the top and bottom segments inwardly overlap top and bottom edges of the arcuate rear segment. By "inwardly overlap" is meant that those segments are radially nearer to the light source and there is an overlap rather than a gap between the segments in the circumferential direction. This design has a rear part further from the light source.

In a third design using these three segments, the arcuate rear segment extends partially around the light source, the top segment inwardly overlaps a top edge of the arcuate rear segment, and the bottom segment outwardly overlaps a bottom edge of the arcuate rear segment. This creates a more turbulent flow path between the two gaps.

In a fourth design using these three segments, the arcuate rear segment extends partially around the light source, the top segment outwardly overlaps a top edge of the arcuate rear segment, and the bottom segment inwardly overlaps a bottom edge of the arcuate rear segment.

The light source for example comprises a flashlamp.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a device for hair removal or treatment of skin;
Fig. 2 shows a typical IPL treatment head; and
Fig. 3 shows a first example of rear reflector design in side view;
Fig. 4 shows a second example of rear reflector design in side view;
Fig. 5 shows a third example of rear reflector design in side view;
Fig. 6 shows a fourth example of rear reflector design in side view; and
Fig. 7 shows the reflector design of Fig. 3 in perspective view.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for hair removal or treatment of skin, comprising a light source for generating treatment light, an output window and a reflector for redirecting output light from the light source to the output window. The reflector is segmented into at least two segments, wherein a gap is provided between the at least two segments. The gap is shielded from a direct light path from the light source by one of the segments. The gap promotes cooling of the light source.

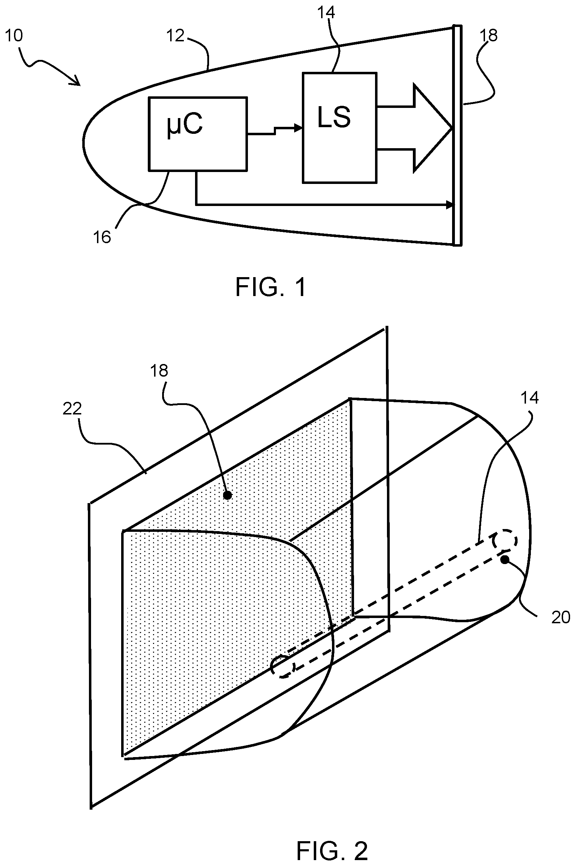

Fig. 1 shows a device 10 for hair removal or treatment of skin, and in particular shows an IPL device as an example. The device comprises an outer housing 12 which contains a flashlamp 14 for generating treatment light in this example. A controller 16 is provided for controlling the light source. The light source delivers treatment light to the skin of the user (and hence to hairs of the user on the skin surface) through an output window 18.

A typical IPL treatment head is illustrated in Fig. 2. The light source 14 for example comprises a Xenon flashlamp. The flashlamp is surrounded by reflecting surfaces, including a curved rear reflector surface 20 which reflects light to the output window 18. The curved reflector shapes the light from the flashlamp into a more or less parallel beam. Reflective ends to the chamber which houses the light source ensure that all light from the Xenon flashlamp reaches the front plate 22 which includes the output window 18, either directly or after one or more reflections.

The light source is typically cylindrical (or it may more generally be a prismatic shape) and it has a length direction which may be considered to correspond to the width direction of the light output window 18.

The closed chamber formed by the reflector does not allow the lamp to cool effectively. It is known to provide holes or a gap at the sides of the reflector to promote cooling, but high temperatures are still experienced particularly in the small gap between the light source and the reflector surface behind it.

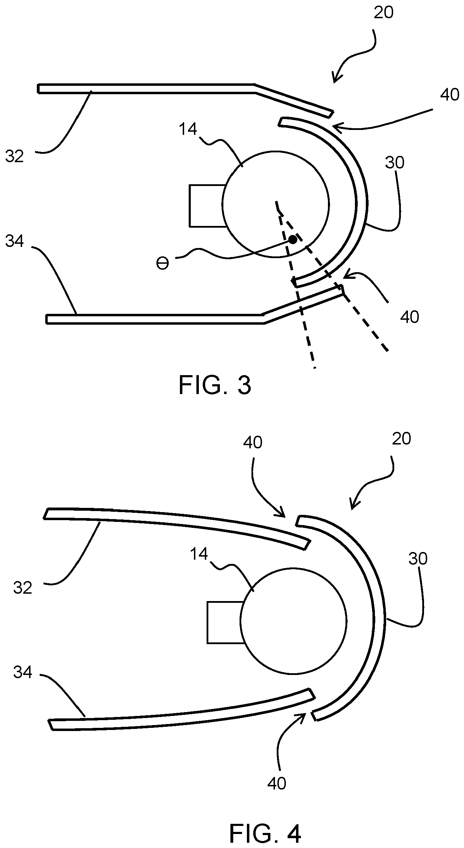

Fig. 3 shows a first example of rear reflector design in side view.

The reflector 20 is segmented into three segments in this example, comprising an arcuate rear segment 30, a top segment 32 and a bottom segment 34. The terms "top" and "bottom" are not intended to require any particular orientation. They are used for ease of understanding, with reference to the orientation shown in Fig. 2.

The segments each extend across the full width of the light output window, so along the full length of the light source. Each segment for example has a constant cross sectional shape along its length, so that the cross section of the reflector is the same at all positions along the width of the light output window, giving uniform light processing properties.

The rear segment partially surrounds the curved cylindrical face of the light source. For example, it curves around at least 90 degrees of the of surface of the light source, for example around between 90 degrees and 180 degrees. The rear segment 30 is the reflecting surface for reflecting rear emitted light from the light source back towards the output window.

The light source typically has a light output which is distributed substantially evenly around its circumference. However, it may have a distribution pattern with an angular characteristic.

There are gaps 40 at the two junctions between the three segments. In this example, the gaps are thus at the top and bottom of the arcuate rear segment 30. The gaps extend along the full length of the light source, and they function as air gaps which allow a flow of cooling air past the light source. The gaps are elongate slits in the structure of the reflector.

The gaps 40 are shielded from a direct light path from the light source so that light cannot escape through the gaps, at least not directly from the light source. There may be light that reaches the gaps after multiple reflections, but the primary light paths from the light source (i.e. with no reflections) are blocked by one of the segments. In particular, there is a region of overlap, when viewed from the direction of a direct light path from the light source. The light source output may be considered to be in a radial direction, and a circumferential overlap, represented by angle Θ in Fig. 3, then ensures there is no direct light escape path. At the limit, Θ may be close to zero.

The gaps 40 allow an airflow in the vicinity of the light source 14, and this airflow has an immediate escape path, rather than a requiring path to the ends of the reflector housing. This promotes more efficient cooling of the light source.

The overlap explained above means the reflection function is not significantly disrupted. The radially inner segment at the region of the overlap acts as a light shield to the gap, in that the light which is directed towards the gap is reflected.

Fig. 3 shows a first design in which the arcuate rear segment 30 extends partially around the rear of the light source, and the top and bottom segments 32, 34 outwardly overlap top and bottom edges of the arcuate rear segment. By "outwardly overlap" is meant that those segments are radially further away from the light source and create the circumferential overlap.

Fig. 4 shows a second design in which the arcuate rear segment 30 extends partially around the rear of the light source, and the top and bottom segments 32, 34 inwardly overlap top and bottom edges of the arcuate rear segment. By "inwardly overlap" is meant that those segments are radially nearer to the light source and create the circumferential overlap.

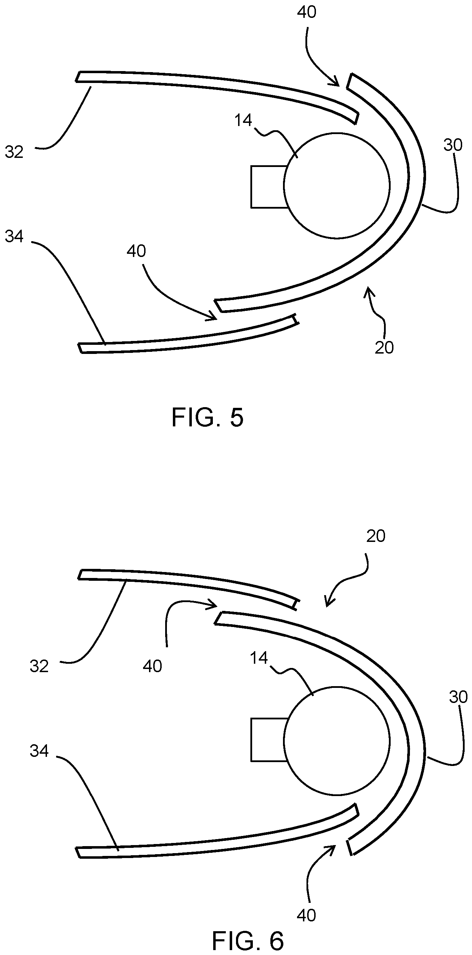

Fig. 5 shows a third design in which the top segment 32 inwardly overlaps a top edge of the arcuate rear segment 30, and the bottom segment 34 outwardly overlaps a bottom edge of the arcuate rear segment 30.

Fig. 6 shows a fourth design in which the top segment 32 outwardly overlaps a top edge of the arcuate rear segment 30, and the bottom segment 34 inwardly overlaps a bottom edge of the arcuate rear segment 30.

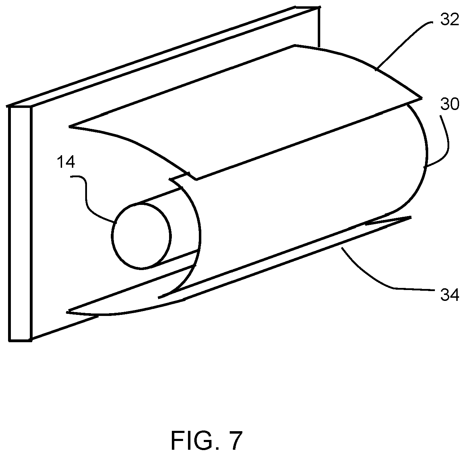

Fig. 7 shows the reflector design of Fig. 3 in perspective view. It shows open ends to the light chamber to show the shapes of the reflector segments, but the ends are preferably closed to provide a light efficient enclosure. The lamp tube is closed at the ends, other than a (sealed) passage for the electrical connection lead, and similarly, the reflector is closed at the ends, but with an opening for the electrical connection lead.

The gaps enable an airflow around the light source to lower the temperature of the light source. This enables light to be generated more efficiently, hence enables a higher light energy and accordingly a faster light treatment speed.

The example above has three segments, but the invention may be applied to a two-segment design.

The invention may be applied to IPL epilators and skin treatment devices. However, it may also be applied to other (optical) epilator designs for example using LEDs or lasers. It may also be applied to other skin treatment devices using light.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device (10) for hair removal or treatment of skin, comprising:
a light source (14) for generating treatment light;
an output window (18) through which the treatment light is delivered to a user's skin, in use; and
a reflector (20) for redirecting output light from the light source (14) to the output window (18),
wherein the reflector is segmented into at least two segments (30, 32, 34), wherein a gap (40) is provided between the at least two segments, wherein the gap is shielded from a direct light path from the light source (25) by one of the segments.

2. The device of claim 1, wherein the light source (14) is prismatic, for example cylindrical, with its length extending along a width direction of the output window (18).

3. The device of claim 1 or 2, wherein the reflector (20) comprises at least three segments (30, 32, 34) and at least two gaps (40) between the segments.

4. The device of claim 3, wherein the reflector (20) comprises an arcuate rear segment (30), a top segment (32) and a bottom segment (34).

5. The device of claim 4, wherein the arcuate rear segment (30) extends partially around the light source(14), and the top and bottom segments (32, 34) outwardly overlap top and bottom edges of the arcuate rear segment.

6. The device of claim 4, wherein the arcuate rear segment (30) extends partially around the light source (14), and the top and bottom segments (32, 34) inwardly overlap top and bottom edges of the arcuate rear segment.

7. The device of claim 4, wherein the arcuate rear segment extends partially around the light source, the top segment inwardly overlaps a top edge of the arcuate rear segment, and the bottom segment outwardly overlaps a bottom edge of the arcuate rear segment.

8. The device of claim 4, wherein the arcuate rear segment (30) extends partially around the light source, the top segment (32) outwardly overlaps a top edge of the arcuate rear segment, and the bottom segment (34) inwardly overlaps a bottom edge of the arcuate rear segment.

9. The device of any one of claims 1 to 8, wherein the light source (14) comprises a flashlamp.
